Europäisches Patentamt

European Patent Office

Office européen des brevets

(19)

(11) EP 0 574 756 B1

(12) EUROPÄISCHE PATENTSCHRIFT

(45) Veröffentlichungstag und Bekanntmachung des Hinweises auf die Patenterteilung:
06.11.1996 Patentblatt 1996/45

(51) Int. Cl.$^6$: **C07C 17/395**, C07C 19/08

(21) Anmeldenummer: 93108578.1

(22) Anmeldetag: 27.05.1993

(54) **Verfahren zur Entfernung olefinischer Verunreinigungen aus fluorierten C3-C6-Kohlenwasserstoffen**

Process for the removal of olefinic impurities from fluorinated C3-C6-hydrocarbons

Procédé pour l'élimination d'impuretés oléfiniques de C3-C6-hydrocarbures fluorés

(84) Benannte Vertragsstaaten:
AT BE CH DE DK ES FR GB GR IE IT LI NL PT SE

(30) Priorität: 13.06.1992 DE 4219414

(43) Veröffentlichungstag der Anmeldung:
22.12.1993 Patentblatt 1993/51

(73) Patentinhaber: HOECHST AKTIENGESELLSCHAFT
65926 Frankfurt am Main (DE)

(72) Erfinder:
• Hopp, Peter, Dr.
W-6238 Hofheim am Ts. (DE)
• Jansen, Rolf-Michael, Dr.
W-6233 Kelkheim/Ts. (DE)

(56) Entgegenhaltungen:
EP-A- 512 502          EP-A- 0 370 688
WO-A-91/06521          DE-B- 1 219 460

# Beschreibung

Die Erfindung betrifft ein Verfahren zur Entfernung olefinischer Verunreinigungen aus fluorierten $C_3$-$C_6$-Kohlenwasserstoffen. Diese Verbindungen sind als Substitute für die ozongefährdenden vollhalogenierten Fluorchlorkohlenwasserstoffe vorgesehen. Um die fluorierten $C_3$-$C_6$-Kohlenwasserstoffe im Bereich der Kältetechnik oder als Treibmittel für Pharma-Aerosole oder Polyurethan-Hartschäume verwenden zu können, müssen störende und giftige olefinische Verunreinigungen, die bei der Synthese - zum Teil nur in Spuren - entstehen, vollständig entfernt werden. Dies ist mit herkömmlichen physikalischen Methoden wie Destillation oder Adsorption in einem wirtschaftlich vertretbaren Rahmen nicht möglich. Deshalb muß nach einer Möglichkeit gesucht werden, die störenden Verunreinigungen durch Reaktion mit geeigneten Substanzen in nicht-toxische Verbindungen umzuwandeln oder in solche, die auf physikalischem Wege mit geringem Aufwand von fluorierten $C_3$-$C_6$-Kohlenwasserstoffen abgetrennt werden können.

In EP-A-370 688 wird diese Abtrennung beschrieben, indem man die verunreinigten - Fluorkohlenwasserstoffe und Fluorhalogenkohlenwasserstoffe bei Temperaturen von bis zu 300 °C mit Kupferoxid, Kobaltoxid, Silberoxid, Mangandioxid oder einer Mischung dieser Stoffe in Kontakt bringt.

Es hat sich nun als günstig herausgestellt, die bei der Herstellung von beispielsweise Isodihydroperfluorhexan (IDPH; 1,1,1,2,3,4,5,5,5-Nonafluor-2-(trifluormethyl)-pentan) entstehenden Olefine, wie 1,1,1,3,4,4,5,5,5-Nonafluor-2-(trifluormethyl)-penten-2 (HFH), mit Alkoholen in Gegenwart von Basen umzusetzen. Hierbei entstehen ausschließlich Verbindungen, die bei der Reindestillation von IDPH leicht abgetrennt werden können. Außerdem wird IDPH nicht angegriffen, und die Reaktion läuft schnell und quantitativ ab.

Die Entfernung olefinischer Verunreinigungen aus 1,1,1,2,3,3,3-Heptafluorpropan (R227) durch Umsetzung mit Alkoholen in Gegenwart von Salzen wird bereits in der EP-A-512 502 beschrieben.

Es ist zwar aus Chemical Abstracts 1971, Vol. 13: 60600w bekannt, daß man aus 2-H-Pentafluorpropen und Alkoholen in Gegenwart von KOH ein Gemisch von höhersiedenden Ethern und Estern herstellen kann. Die Umsätze sind jedoch nicht quantitativ. Daher war nicht zu erwarten, daß man Spuren von HFH und anderen Olefinen, gelöst in IDPH, durch Umsetzung mit Alkoholen quantitativ entfernen kann. Außerdem war eine Azeotropbildung mit Alkoholen zu befürchten.

Gegenstand der Erfindung ist ein Verfahren zur Entfernung olefinischer Verunreinigungen der allgemeinen Formel $C_nH_mF_pCl_q$, wobei n = 2 - 6, m = 0 - 8, p = 1 - 12, q = 0 - 8 und m + p + q = 2n ist, aus fluorierten $C_3$-$C_6$-Kohlenwasserstoffen, mit Ausnahme von 1,1,1,2,3,3,3-Heptafluorpropan (R 227), das dadurch gekennzeichnet ist, daß man die verunreinigten fluorierten $C_3$-$C_6$-Kohlenwasserstoffe, mit einem Alkohol und einer Base bei Temperaturen von -20 bis 100°C und Drücken von 1 bis 50 bar in Kontakt bringt und gleichzeitig oder anschließend die fluorierten $C_3$-$C_6$-Kohlenwasserstoffe abdestilliert.

Vorzugsweise setzt man als fluorierten $C_3$-$C_6$-Kohlenwasserstoff 1,1,1,3-Tetrafluorpropan, 1,1,1,4,4,4-Hexafluorbutan, 1,1,1,2,3,4,4,4-Oktafluorbutan, 1,1,2,2,3,3,4,4-Oktafluorbutan oder 1,1,1,2,3,4,5,5,5-Nonafluor-2-(trifluormethyl)-pentan ein.

Vorzugsweise verwendet man einen Alkohol der allgemeinen Formel $C_aH_{2a+1}OH$, $C_aH_{2a}(OH)_2$, $C_aH_{2a-1}OH$ oder $C_aH_{2a-2}(OH)_2$, mit a = 1 bis 6, insbesondere mit a = 1 bis 3. Besonders geeignet sind Methanol, Ethanol, i-Propanol und Ethylenglykol (Glykol).

Als Basen verwendet man vorzugsweise solche, deren pK-Wert bei 8 bis 14 liegt, insbesondere NaOH, KOH oder Na-Phenolat.

Die Temperatur liegt vorzugsweise bei 0 bis 50°C, und der Druck beträgt vorzugsweise 1 bis 10 bar. Bezogen auf fluorierten $C_3$-$C_6$-Kohlenwasserstoff beträgt die Menge an Alkohol vorzugsweise 0,5 bis 20 Gew.-%, insbesondere 0,5 bis 10 Gew.-%, und die Menge an Base beträgt vorzugsweise 0,01 bis 10 Gew.-%, insbesondere 0,01 bis 5 Gew.-%.

Die Erfindung soll anhand der folgenden Beispiele näher erläutert werden.

Beispiel 1 bis 9:

Ein beheizbarer Rührautoklav (V = 300 ml) wurde mit 100 g Isodihydroperfluorhexan (IDPH), das ca. 500 ppm 1,1,1,3,4,4,5,5,5-Nonafluor-2-(trifluormethyl)-penten-2 (HFH) enthielt, sowie mit 10 g eines Alkohols und 1 g einer Base beschickt. Anschließend wurde der Autoklav auf 50° C erwärmt und 8 Stunden bei dieser Temperatur gerührt. Der Gehalt an HFH wurde gaschromatographisch verfolgt. Die eingesetzten Alkohole und Basen und die Ergebnisse sind in Tabelle 1 dargestellt.

Tabelle 1

| Alkohol | Base | Gehalt HFH [ppm] nach 8 Stunden |
|---------|------|-------------------------------|
| Methanol | NaOH | u.N.* |
| Methanol | KOH | u.N.* |
| Methanol | Na-Acetat | 380 |
| Methanol | $Na_2HPO_3$ | 450 |
| Methanol | Pyridin | 280 |
| Methanol | Na-Phenolat | 50 |
| Ethanol | NaOH | u.N.* |
| i-Propanol | NaOH | 40 |
| Glykol | NaOH | u.N.* |

*u.N. = unter Nachweisgrenze (<1 ppm)

Beispiel 10:

Ein Destillationsgefäß wurde mit 80 kg IDPH, das ca. 500 ppm HFH enthielt, sowie mit 800 g Methanol und 50 g KOH beschickt. Anschließend wurde das Gefäß auf 40° C erwärmt und 12 Stunden bei dieser Temperatur gerührt. Der Gehalt an HFH war danach auf weniger als 10 ppm gefallen.

## Patentansprüche

1. Verfahren zur Entfernung olefinischer Verunreinigungen der allgemeinen Formel $C_nH_mF_pCl_q$, wobei n = 2 - 6, m = 0 - 8, p = 1 - 12, q = 0 - 8 und m + p + q = 2n ist, aus fluorierten $C_3$-$C_6$-Kohlenwasserstoffen, mit Ausnahme von 1,1,1,2,3,3,3-Heptafluorpropan (R 227), dadurch gekennzeichnet, daß man die verunreinigten fluorierten $C_3$-$C_6$-Kohlenwasserstoffe mit einem Alkohol und einer Base bei Temperaturen von -20 bis 100° C und Drucken von 1 bis 50 bar in Kontakt bringt und gleichzeitig oder anschließend die fluorierten $C_3$-$C_6$-Kohlenwasserstoffe abdestilliert.

2. Verfahren nach Anspruch 1, dadurch gekennzeichnet, daß man als fluorierten $C_3$-$C_6$-Kohlenwasserstoff 1,1,1,3-Tetrafluorpropan, 1,1,1,4,4,4-Hexafluorbutan, 1,1,1,2,3,4,4,4-Oktafluorbutan, 1,1,2,2,3,3,4,4-Oktafluorbutan oder 1,1,1,2,3,4,5,5,5-Nonafluor-2-(trifluormethyl)-pentan einsetzt.

3. Verfahren nach Anspruch 1 oder 2, dadurch gekennzeichnet, daß man einen Alkohol der allgemeinen Formel $C_nH_{2n+1}OH$, $C_nH_{2n}(OH)_2$, $C_nH_{2n-1}OH$ oder $C_nH_{2n-2}(OH)_2$ mit n = 1 - 6, vorzugsweise n = 1 - 3 verwendet.

4. Verfahren nach einem der Ansprüche 1 bis 3, dadurch gekennzeichnet, daß man eine Base verwendet, deren pK-Wert bei 8 bis 14 liegt.

5. Verfahren nach einem der Ansprüche 1 bis 4, dadurch gekennzeichnet, daß man bei 0 bis 50°C und 1 bis 10 bar arbeitet.

6. Verfahren nach einem der Ansprüche 1 bis 5, dadurch gekennzeichnet, daß man als Alkohol Ethylenglykol, Methanol, Ethanol oder i-Propanol verwendet.

7. Verfahren nach einem der Ansprüche 1 bis 6, dadurch gekennzeichnet, daß man als Base NaOH, KOH oder Na-Phenolat verwendet.

8. Verfahren nach einem der Ansprüche 1 bis 7, dadurch gekennzeichnet, daß man 0,5 bis 20 Gew.-% Alkohol und 0,01 bis 10 Gew.-% Base verwendet, bezogen auf den fluorierten $C_3$-$C_6$-Kohlenwasserstoff.

## Claims

1. A process for removing olefinic impurities of the formula $C_nH_mF_pCl_q$, where n = 2 - 6, m = 0 - 8, p = 1 - 12, q = 0 - 8 and m + p + q = 2n, from fluorinated $C_3$-$C_6$-hydrocarbons, with the exception of 1,1,1,2,3,3,3-heptafluoropropane (R 227), which comprises bringing the contaminated fluorinated $C_3$-$C_6$-hydrocarbons into contact with an alcohol and a base at temperatures of -20 to 100°C and pressures of 1 to 50 bar and, simultaneously or subsequently, distilling off the fluorinated $C_3$-$C_6$-hydrocarbons.

2. The process as claimed in claim 1, wherein the fluorinated $C_3$-$C_6$-hydrocarbon used is 1,1,1,3-tetrafluoropropane, 1,1,1,4,4,4-hexafluorobutane, 1,1,1,2,3,4,4,4-octafluorobutane, 1,1,2,2,3,3,4,4-octafluorobutane or 1,1,1,2,3,4,5,5,5-nonafluoro-2-(trifluoromethyl)-pentane.

3. The process as claimed in claim 1 or 2, wherein an alcohol of the formula $C_nH_{2n+1}OH$, $C_nH_{2n}(OH)_2$, $C_nH_{2n-1}OH$ or $C_nH_{2n-2}(OH)_2$ where n = 1 - 6, preferably n = 1 - 3, is used.

4. The process as claimed in one of claims 1 to 3, wherein a base is used whose pK is 8 to 14.

5. The process as claimed in one of claims 1 to 4, wherein the procedure is carried out at 0 to 50°C and 1 to 10 bar.

6. The process as claimed in one of claims 1 to 5, wherein the alcohol used is ethylene glycol, methanol, ethanol or i-propanol.

7. The process as claimed in one of claims 1 to 6, wherein the base used is NaOH, KOH or Na phenolate.

8. The process as claimed in one of claims 1 to 7, wherein 0.5 to 20% by weight of alcohol and 0.01 to 10% by weight of base are used, based on the fluorinated $C_3$-$C_6$-hydrocarbon.

## Revendications

1. Procédé pour l'élimination des impuretés oléfiniques de formule générale $C_nH_mF_pCl_q$ où n = 2 - 6, m = 0 - 8, p = 1 - 12, q - 0 - 8 et m + p + q = 2n, à partir des hydrocarbures en $C_3$-$C_6$ fluorés à l'exception du 1,1,1,2,3,3,3-heptafluoropropane (R 227), caractérisé en ce qu'on met en contact les hydrocarbures en $C_3$-$C_6$ fluorés souillés avec un alcool et une base, à des températures de -20 à 100°C, et des pressions de 1 à 50 bars, et simultanément ou après, on sépare par distillation les hydrocarbures en $C_3$-$C_6$ fluorés.

2. Procédé selon la revendication 1, caractérisé en ce qu'on utilise en tant d'hydrocarbure en $C_3$-$C_6$ fluoré le 1,1,1,3-tétrafluoropropane, le 1,1,1,4,4,4-hexafluorobutane, le 1,1,1,2,3,4,4,4-octafluorobutane, le 1,1,2,2,3,3,4,4-octafluoro-butane ou le 1,1,1,2,3,4,5,5,5-nonafluoro-2-(trifluorométhyl)-pentane.

3. Procédé selon la revendication 1 ou 2, caractérisé en ce qu'on utilise un alcool de formule générale $C_nH_{2n+1}OH$, $C_nH_{2n}(OH)_2$, $C_nH_{2n-1}OH$ ou $C_nH_{2n-2}(OH)_2$ avec n = 1 - 6, de préférence n = 1 - 3.

4. Procédé selon l'une des revendications 1 à 3, caractérisé en ce qu'on utilise une base dont la valeur du pK est de 8 à 14.

5. Procédé selon l'une des revendications 1 à 4, caractérisé en ce qu'on travaille à une température de 0 à 50°C et une pression de 1 à 10 bars.

6. Procédé selon l'une des revendications 1 à 5, caractérisé en ce qu'on utilise en tant qu'alcool l'éthylèneglycol, le méthanol, l'éthanol ou l'i-propanol.

7. Procédé selon l'une des revendications 1 à 6, caractérisé en ce qu'on utilise en tant que base NaOH, KOH ou phénolate de Na.

8. Procédé selon l'une des revendications 1 à 7, caractérisé en ce qu'on utilise de 0,5 à 20 % en poids d'alcool et de 0,01 à 10 % en poids de base par rapport à l'hydrocarbure en $C_3$-$C_6$ fluoré.